Europäisches Patentamt

European Patent Office

Office européen des brevets

(19)

(11) Publication number: **0 106 604**
**B1**

(12) **EUROPEAN PATENT SPECIFICATION**

(45) Date of publication of patent specification: **09.08.89**

(51) Int. Cl.⁴: **D 04 H 1/54**

(21) Application number: **83305891.0**

(22) Date of filing: **29.09.83**

(54) Patterned belt bonded material and method for making the same.

(30) Priority: **30.09.82 US 430309**

(43) Date of publication of application:
**25.04.84 Bulletin 84/17**

(45) Publication of the grant of the patent:
**09.08.89 Bulletin 89/32**

(84) Designated Contracting States:
**AT BE CH DE FR IT LI LU NL SE**

(56) References cited:
**DE-A-2 644 961**
**FR-A-1 059 617**
**US-A-3 616 031**
**US-A-4 103 058**

(73) Proprietor: **CHICOPEE**
**317 George Street**
**New Brunswick New Jersey 08903 (US)**

(72) Inventor: **Mays, Alfred T.**
**6 Lynnfield Drive**
**East Windsor New Jersey 08520 (US)**

(74) Representative: **Jones, Alan John et al**
**CARPMAELS & RANSFORD 43 Bloomsbury**
**Square**
**London, WC1A 2RA (GB)**

Courier Press, Leamington Spa, England.

**Description**

Background of the Invention

Honeycomb currently markets an apparatus for heating and fusing thermoplastic fibers in a web. The apparatus is a modification of their thru-air bonder for drying and heat setting fibrous webs. In this apparatus, the web is carried about a rotating drum and heated air is blown onto the web. When starting with a high loft, low density web, such an apparatus yields a very high loft, low density fabric having limited strength. Our copending European Patent Application No. 0 105 730 discloses an apparatus and method wherein the web is carried about an open mesh structure which is substituted for the surface of the drum. This apparatus and method produces a unique fabric having patterned regions of even higher loft where fibers are bent out of the plane of the fabric. Another method and apparatus for heat fusing thermoplastic fibers in a web set forth in our copending European Patent Application No. 0 105 731, the web is picked up by a first carrier belt, a second carrier belt is placed on top of the web and the double belt laminate is passed about heated rolls. A fabric made by the double belt bonder has good strength including good cross directional strength but is more dense and not as lofty as fabric made on the Honeycomb apparatus.

Summary of the Invention

The present invention comprises a method and apparatus for forming a fabric from a web comprising at least five percent low shrink thermoplastic fibers and the fabric formed thereby. As used herein, the term "thermoplastic fibers" encompasses conjugate fibers. The method comprises heating a web comprising said low shrink thermoplastic fibers to heat fuse the fibers in the web while the web is tensioned between a first carrier belt and an open mesh belt. When conjugate fibers are used, the method comprises fusing at least the low melting point component of the conjugate fibers. The apparatus of the present invention comprises heating means, a first carrier belt and means for causing said belt to travel in an arcuate path adjacent the heating means, an open mesh belt and means for causing said open mesh belt to travel along an initial path spaced from said first carrier belt and then along said arcuate path. The fabric formed by the method and apparatus of the present invention has a surface pattern on at least one surface thereof and patterned densified regions which are created by the open mesh belt. The fabric has high loft and low density as well as good strength. The open mesh belt may be overlaid by a second carrier belt sandwiching the web and open mesh belt between two carrier belts. The first carrier belt may comprise a second open mesh belt to form a fabric with a pattern on both surfaces. The strength, cushioning, and absorbency of the fabric make it attractive for many end uses including as a diaper facing material. When used as a diaper facing, the fabric may comprise hydrophobic fibers. When only one surface of the fabric is patterned, the fabric displays a greater porosity on the patterned side and a lesser porosity on the unpatterned side providing enhanced wicking from the patterned surface and minimize strike back when used as, for example, a diaper facing.

Brief Description of the Drawings

Figure 1 is a schematic side view of one embodiment of the apparatus and method to the present invention.

Figure 2 is a plan view of a portion of the open mesh belt for use according to the present invention.

Figure 3 is a perspective view of one embodiment of a fabric according to the present invention.

Figure 3A is a fragmentary section of the fabric of Figure 3 illustrating schematically how the fabric was formed.

Figure 4 is a perspective view of another embodiment of a fabric according to the present invention.

Figure 4A is a fragmentary section of the fabric of Figure 4 illustrating schematically how the fabric was formed.

Description of the Drawings

The present invention comprises an apparatus and a method for making a fabric from a web comprising low shrink thermoplastic fibers, which may be conjugate fibers, and the fabric formed thereby. It is preferred to employ sheath/core conjugate fibers of polyethylene/polyester respectively. Sheath/core fibers of polyethylene/polypropylene may also be used, as well as low shrink thermoplastic such as Eastman 4BC crystalline copolyester monofil. Either eccentric or concentric sheath/core fibers can be employed.

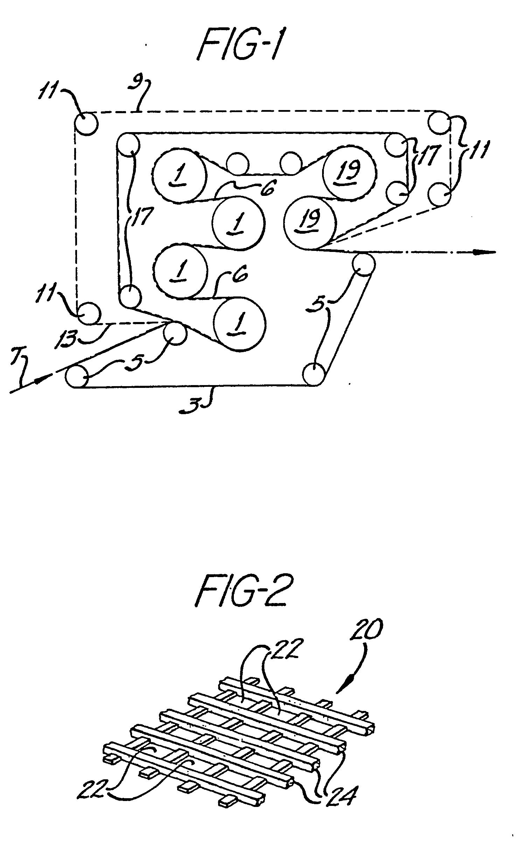

Figure 1 illustrates, schematically, a side view of one embodiment of the apparatus of the present invention wherein the heating means comprises heated rolls or steam cans. Alternate heating means, such as a Honeycomb apparatus, may be used, including any other heating means where the belts can travel in tensioned arcuate paths adjacent the heating means.

In the method shown, a first endless carrier belt 3, is provided having means 5, for causing said first endless carrier belt to travel in a curvilinear path 6, adjacent the heating means. Web 7 is superimposed on said first carrier belt 3. In its broadest aspect, the present invention includes the surface of a Honeycomb type dryer as the first endless carrier belt.

According to the present invention, an open mesh belt 9 is superimposed on the web prior to heating. The open mesh structure may comprise an endless belt provided with means 11 for causing said open mesh belt to travel along an initial path 13 spaced from said first endless carrier belt, and then along the curvilinear path 6 around the heating means. A second endless carrier belt 15 may be superimposed on top of the open mesh structure 9. When a second endless carrier belt is used, the open mesh belt may comprise belt segments. The second endless carrier belt is provided with means 17 for causing said belt to travel along path 6. The conjugate fibers in the web, when heated, fuse to fibers in the web to provide a strong, high loft, low density fabric.

In a preferred embodiment of the method of the present invention the web is cooled before it is separated from the first carrier belt and the open mesh belt. To facilitate cooling, the belts may be passed around at least one cooling roll shown at 19 in Figure 1. By maintaining the web between the belts until the web is cooled, the method avoids the loss in cross directional strength due to drafting as a web is removed from a roll on one carrier belt. Also the use of the two belts prevents sticking of the heated thermoplastic fibers of the web to the rolls and permits rapid handling of the web while heated, including immediately passing the web about cooling rolls.

Figure 2 illustrates a portion of an open mesh belt 20 used in the present invention. The open areas 22 and depth 24 of the open mesh belt allow the web to maintain its high loft and low density through the heat bonding procedure. The depth 24 of the open mesh belt maintains the distance between the tensioned first and second carrier belts so that the web is not crushed therebetween. The depth of the open mesh belt and the degree of open area 22 determine the loft of the final fabric material.

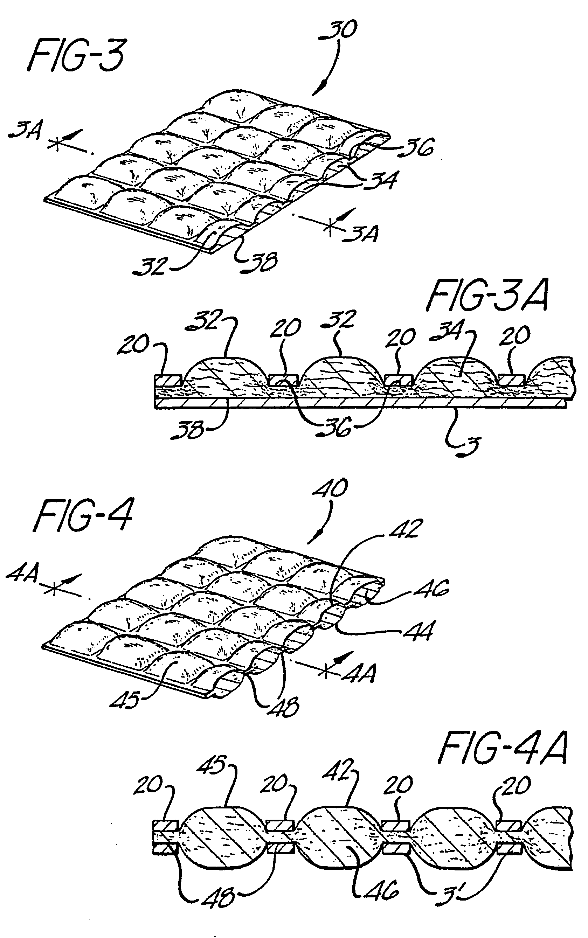

Figure 3 illustrates a fabric formed according to the method of the present invention. As shown in Figure 3A, the fabric is formed using one open mesh structure as seen in Figure 2. The fabric shown generally at 30 has one surface thereof 32 which is patterned. The surface 32 consists of regions 34 of high loft, low density and good porosity and regions 36 that are densified between the open mesh structure 20 and carrier belt 3. Surface 38 formed against belt 3 is smooth. The fabric has a porosity differential from the surface 32 to the opposite surface 38 and hence may be used advantageously in many applications, for instance, as a diaper facing fabric. The fabric formed according to the method of the present invention exhibits a high loft, lower density and better strength than fibers formed on a double belt bonder as described in copending application EP—A—0 105 731 or with a thru-air bonder.

In the following example, samples of a web formed with a dual rotor were fusion bonded according to the methods listed.

Example 1

| | Thru-air | Present Invention | Double-Belt |
|---|---|---|---|
| Weight oz/yd.² (g/m²) | 3.0 (101.7) | 3.1 (105.1) | 3.2 (108.5) |
| Bulk inch (mm) | .066 (1.68) | .060 (1.52) | .042 (1.07) |
| Density gm/cm³ | .0613 | .0694 | .1002 |
| Tensile lb.(N) | 1.0 (4.45) | 1.5 (6.67) | 1.8 (8.01) |
| Tenacity lb./inch/100 grains N/m/10 g | .08 (21.6) | .11 (29.7) | .13 (35.1) |
| Absorbency % | 1329 | 1155 | 896 |
| Rate sec | 3.1 | 3.1 | 9.9 |
| Softness MD gram | 135.5 | 133 | 162.7 |
| CD gram | 88.1 | 112 | 162.8 |

As can be seen, the fabric formed according to the present invention maintains a high bulk and low density similar to that of the thru-air bonder but has a strength approximately that of a double belt bonded fabric.

Webs used in the present invention may be prepared by air laying or carding of the fibers. The weight of the web may range from 0.3 to 5 ozs./yd. (10.2 to 169.5 g/m²). The present invention preferably utilizes a conjugate fiber with an outer sheath of thermoplastic material. In a most preferred embodiment, the fiber is capable of self-crimping when heated and the method involves heating to effect autogenous bonding of the web and self-crimping of the thermoplastic fibers. The web may further comprise synthetic wood pulp, wood pulp, cellulosic or other fibers. The percentage of the low shrink thermoplastic fibers in the web may vary from five to 100 percent.

The open mesh belt is preferably formed of a material with easy release properties and/or good heat transmission. The structure of the open mesh belt may vary according to the desired properties of the fabric; the more open mesh yielding more bulk, softness and absorbency; the tighter mesh yielding higher tensile strength as set forth in the following Example.

## Example 2

| Mesh [wiresperinch (percm)] | 3.0 (1.2) | 4.7 (1.9) | 5.3 (2.1) | 5.8 (2.3) | 6.0 (2.4) | 6.8 (2.7) |
|---|---|---|---|---|---|---|
| Weight oz/yd.$^2$ (g/m$^2$) | 3.1 (105.1) | 3.2 (108.5) | 3.1 (105.1) | 3.3 (111.9) | 3.1 (105.1) | 3.2 (108.5) |
| Bulk inch | .060 | .062 | .060 | .058 | .053 | .056 |
| Density gm/cm$^3$ | .0694 | .0682 | .0694 | .0758 | .0786 | .0756 |
| Tensile MD lb. (N) | 1.5 (6.67) | 1.7 (7.56) | 1.5 (6.67) | 1.8 (8.01) | 1.8 (8.01) | 1.8 (8.01) |
| Tenacity lb/ inch/100 grains (N/m/10 g) | .11 (29.7) | .12 (32.4) | .11 (29.7) | .12 (32.4) | .13 (35.1) | .13 (35.1) |
| Absorbency % | 1155 | 1219 l | 1155 | 1070 | 1038 | 1062 |
| Rate sec | 3.1 | 2.9 | 3.1 | 4.0 | 3.8 | 4.3 |
| Softness: MD gram | 133 | 162.6 | 132.7 | 143.9 | 154.9 | 152.1 |
| CD gram | 112 | 109.5 | 112.4 | 138.6 | 123.1 | 129.7 |

Figure 4 illustrates another fabric formed by the method and apparatus of the present invention. As shown in Figure 4A, another open mesh belt is substituted for the first carrier belt or interposed between the first carrier belt and the web. The fabric is shown generally at 40. Both surfaces 42 and 44 of the fabric have a surface pattern. As the surfaces 42 and 44 are formed by means of separate open mesh belts 3' and 9, they need not have the same pattern and may be formed by different open mesh belts and belts out of registry. Each of the surfaces 42 and 44 have regions 46 of high bulk and low density and good porosity and regions 48 that are densified. Though the fabrics made according to the method of the present invention have many uses, they are particularly suited for those end uses requiring strength, bulk and absorbency.

### Claims

1. Process for making a nonwoven fabric having high bulk, low density, and good strength comprising:
(a) superimposing a web comprising at least 5% low shrink thermoplastic fibers on a first carrier belt;
(b) superimposing an open mesh belt on the web to form a two belt laminate containing said web; and
(c) causing the laminate to travel in a curvilinear path adjacent a heating means to heat fuse said thermoplastic fibers to adjacent fibers in the web; and
(d) cooling said web, whereby a strong, high bulk, low density web is formed having a patterned surface adjacent said open mesh belt.

2. The process of Claim 1 wherein said first carrier belt comprises a second open mesh belt.

3. The process of Claim 1 or 2 further comprising the step of:
superimposing a second carrier belt on the open mesh to sandwich the web and open mesh belt or belts between two carrier belts before heating the web.

4. The process of Claim 1 wherein said thermoplastic fibers comprise conjugate fibers having the thermoplastic component on the outer surface thereof.

5. Apparatus for heat fusing low shrink thermoplastic fibers in a nonwoven web, said apparatus comprising heating means, a first endless carrier belt and means for causing the first endless carrier belt to travel in a curvilinear path adjacent the heating means, an open mesh belt and means for causing the open mesh belt to travel along an initial path spaced from said first belt and then along said curvilinear path.

6. An apparatus as in Claim 5 further comprising means for cooling said web and belts.

7. An apparatus as in Claim 6 wherein said means for cooling comprises at least one cooling roll.

### Patentansprüche

1. Verfahren zur Herstellung eines Faservliesstoffes von hoher Bauschigkeit, niedriger Dichte und guter Festigkeit, welches Verfahren die folgenden Schritte umfaßt:
(a) das Auflegen einer Bahn, welche wenigstens 5% thermoplastische Fasern mit geringen Schrumpfungseigenschaften enthält, auf einem ersten Trägerband;

4

(b) das Auflegen eines offenmaschigen Bandes auf der Bahn unter Bildung eines zwei Bänder umfassenden und die Bahn enthaltenden Laminates; und

(c) das Führen des Laminates auf einem krummlinigen Weg neben einer Heizeinrichtung vorbei, um die thermoplastischen Fasern mit benachbarten Fasern in der Bahn warmzuverschweißen; und

(d) das Abkühlen der Bahn, wobei eine feste, hochvoluminöse Bahn von niedriger Dichte gebildet wird, welche neben dem offenmaschigen Band eine gemusterte Oberfläche aufweist.

2. Verfahren nach Anspruch 1, wobei das erste Trägerband ein zweites offenmaschiges Band umfaßt.

3. Verfahren nach Anspruch 1 oder 2, welches weiterhin die folgende Stufe umfaßt:

das Auflegen eines zweiten Trägerbandes auf dem offenmaschigen Band, um die Bahn und das offenmaschige Band oder die offenmaschigen Bänder vor dem Erhitzen der Bahn zwischen zwei Trägerbändern zu einem sandwichartigen Gebilde zu vereinigen.

4. Verfahren nach Anspruch 1, wobei die thermoplastischen Fasern Konjugatfasern umfassen, welche die thermoplastische Komponente auf der Außenseite der Konjugatfasern aufweisen.

5. Vorrichtung zum Warmverschweißen von thermoplastischen Fasern mit geringen Schrumpfungseigenschaften in einer Faservliesstoffbahn, wobei diese Vorrichtung eine Heizeinrichtung, ein erstes Endlosträgerband sowie Einrichtungen, welche dazu dienen, das erste Endlosträgerband in einen krummlinigen Weg neben der Heinzeinrichtung zu führen, ein offenmaschiges Band sowie Einrichtungen umfaßt, welche dazu dienen, das offenmaschige Band auf einem anfänglichen Weg zu führen, der sich in einem Abstand von dem ersten Band befindet, und dann längs des krummlinigen Weges zu führen.

6. Vorrichtung nach Anspruch 5, welche weiterhin Einrichtungen zum Abkühlen der Bahn und der Bänder umfaßt.

7. Vorrichtung nach Anspruch 6, worin die Einrichtung zum Abkühlen wenigstens eine Kühlwalze umfaßt.

## Revendications

1. Procédé de production d'un textile non tissé ayant un volume élevé, une faible densité et une bonne résistance qui comprend:

(a) la superposition d'une nappe comprenant au moins 5% de fibres thermoplastiques à faible rétrécissement sur une première courroie porteuse;

(b) la superposition d'une courroie à maille ouverte sur la nappe pour former un lamifié de deux courroies comprenant ladite nappe; et

(c) la mise en circulation du lamifié sur un chemin curviligne adjacent à des moyens de chauffage pour chauffer et fondre lesdites fibres thermoplastiques sur les fibres adjacentes de la nappe; et

(d) le refroidissement de ladite nappe, ce qui permet d'obtenir une nappe solide, de volume élevé, de faible densité, ayant une surface gaufrée adjacente à ladite courroie à maille ouverte.

2. Procédé selon la revendication 1, dans lequel la. première courroie porteuse comprend une deuxième courroie à maille ouverte.

3. Procédé selon la revendication 1 ou 2, qui comprend en plus l'étape de:

superposition d'une deuxième courroie porteuse sur la courroie à maille ouverte pour prendre en sandwich la nappe et la courroie ou les courroies à maille ouverte entre deux courroies porteuses avant de chauffer la nappe.

4. Procédé selon la revendication 1, dans lequel lesdites fibres thermoplastiques comprennent des fibres complexes ayant le composant thermoplastique à la surface externe.

5. Appareil pour souder à la chaleur des fibres thermoplastiques à faible rétrécissement incorporées dans une nappe non tissée, ledit appareil comprenant des moyens de chauffage, une première courroie porteuse sans fin et des moyens pour entraîner la première courroie porteuse sans fin dans un trajet curviligne adjacent aux moyens de chauffage, une courroie à maille ouverte et des moyens pour entraîner la courroie à maille ouverte le long d'un trajet initialement écarté de celui de la première courroie, puis le long dudit trajet curviligne.

6. Appareil selon la revendication 5, comprenant en plus, des moyens de refroidissement desdites nappe et courroies.

7. Appareil selon la revendication 6, dans lequel lesdits moyens de refroidissement comprennent au moins un cylindre de refroidissement.

## FIG-1

## FIG-2

FIG-3

FIG-3A

FIG-4

FIG-4A